# EUROPEAN PATENT SPECIFICATION

(11) **EP 2 659 895 B1**
(45) Date of publication and mention of the grant of the patent: **23.08.2017**
(21) Application number: 11820891.7
(22) Date of filing: 21.12.2011
(51) Int. Cl.: A61K 31/728, A61K 31/715, A61K 9/20

(54) **PHARMACEUTICAL COMPOSITION INCLUDING HYALURONIC ACID AND A ZINC COMPOUND AND CORRESPONDING BAR ON A STICK AND PREPARATION METHOD**
PHARMAZEUTISCHE ZUSAMMENSETZUNG MIT HYALURONSÄURE UND EINER ZINKVERBINDUNG SOWIE ENTSPRECHENDE TABLETTE MIT STÄBCHEN UND HERSTELLUNGSVERFAHREN
COMPOSITION PHARMACEUTIQUE À BASE D'ACIDE HYALURONIQUE ET D'UN COMPOSÉ DE ZINC ET COMPRIMÉ AVEC BÂTONNET ET PROCÉDÉ DE PRÉPARATION ASSOCIÉ

(30) Priority: 27.12.2010 ES 201031958
(43) Date of publication of application: 06.11.2013
(73) Proprietor: LABORATORIOS VIÑAS S.A., 08025 Barcelona (ES)
(72) Inventor: BUXADÉ VIÑAS, Antonio, E-08036 Barcelona (ES); CONCHILLO TERUEL, Antonio, E-08025 Barcelona (ES); JULIÁN PRAT, Ramón, E-08025 Barcelona (ES)
(74) Representative: Curell Suñol S.L.P.
(86) International application number: PCT/ES2011/070892
(87) International publication number: WO 2012/089882

(56) References cited:
- WO-A1-98/48815
- WO-A1-2010/121081
- JP-A- 2002 029 950
- US-A1- 2004 037 788

## Description

### Field of the invention

The present invention relates to a pharmaceutical composition in the form of a tablet with stick containing hyaluronic acid and a zinc compound for the treatment of buccal ulcers in children and a to process for the preparation thereof.

### State of the Art

Buccal ulcers are pathologies of the buccal mucosa that appear both in children and in adults. Most of the ulcers correspond to herpes, to aphthas and irritation caused by accidental bites, burns caused by hot meals or drinks, contact with corrosive or toxic substances, dental correction apparatus, etc. They are also one of the most frequent and painful diseases in the buccal mucosa, with numerous preparations presented in very diverse pharmaceutical forms: gels, sprays, sticks, mouthwash solutions, orally dissolvable tablets, ointments, tinctures, extracts, etc., having been used for the treatment thereof.

Topical applications have been used for centuries in preventive, curative, or symptomatic treatment, by means of different forms of use, composition and application. Thus, numerous drugs that contain steroidal and non-steroidal anti-inflammatory drugs, antibiotics, local anaesthetics, disinfectants, etc. are available

In the bibliography on treatments of buccal ulcers two important approaches can be found: the therapeutical compositions to be applied in relation to the active ingredient to be used; and the vehicle used to apply the active ingredient on which the variety of galenic formulations used is outstanding.

Among the various active ingredients used, the use of steroidal or non-steroidal anti-inflammatory drugs, as well as antiseptics and local anaesthetics is outstanding. Also various formulations containing hyaluronic acid or derivatives thereof and to a lesser degree zinc salts are to be found.

Hyaluronic acid is a natural polysaccharide of the glycosaminoglycan type composed of alternate residues of D-glycuronic acid and N-acetyl-D-glucosamine, having a structural function. It is a linear polymer with a molecular weight, depending on the source and the methods of preparation, of between 50,000 and 15,000,000 Daltons. It is present in all living organisms and is a universal component of the extracellular space in tissues, constituting a matrix that allows lubrication, absorption, transportation of the nutrients to the cells and elimination of the waste therefrom. Hyaluronic acid plays a decisive role as a conjunctive tissue element, being an essential element in the skin, with approximately 50% of the hyaluronic acid concentration existing in the body. It is abundant in the vitreous humour, in the sinovial liquid, the umbilical cord tissue, as well as in skin connective tissues, with functions of contribution of elasticity, development of collagen and water retention. In relation to its effectiveness, a very important aspect, extensively investigated and documented on hyaluronic acid, is the development of a wide range of galenic formulations and compositions that are part of the regular vehicles used in the cosmetic and therapeutical fields. Thus for example, liquid, solid or semisolid topical preparations in form of oil/water (O/W) and water in oil (W/O) emulsions have been used, ointments and salves, pastes, gels, solutions, suspensions, dispersions, powders, mouth washes, sprays, etc.

On the other hand, zinc is an essential trace element in the human body and its role as immunonutrient, cofactor in protein synthesis and in the healing of wounds by promoting epithelialization, as well as its intervention in the resistance to epithelial apoptosis, to cytoproteccion and against bacterial toxins and therefore, with anti-infectious action, is known. Also it seems that the application and topical action of zinc in the form of oxides or salts is superior to oral therapy (Lansdown et al. Wound Rep. Reg. 2007; 15: 2-16).

The bibliography cited hereinafter is a sample of the present knowledge in relation to these active ingredients and their forms of application in the treatment of buccal ulcerations.

Document ES 2236324 describes a hyaluronic acid based aqueous liquid together with glyciretinic acid and polyvinylpyrrolidone for the treatment of typical ulcerous and inflammatory disorders of humid surfaces, among them the buccal mucosa.

In document ES 2080844 there are described different formulations: gingival pastes, toothpastes, adhesive pastes and powders, rinsing solutions, etc. for the treatment of inflammation and the hygiene and cosmetic treatment of the oral cavity.

In document ES 2314304 there are disclosed topical compositions in the form of gels, creams or pastes containing hyaluronic acid in combination with methyl-sulfonyl-methane and malaleuca alternifolia extract for the prevention and treatment of processes of the membrane of the buccal mucosa among other mucous membranes.

WO 2005/000321 describes pharmaceutical compositions in the form of O/W or W/O emulsions, ointments, creams, pastes, gels, suspensions, powders, dispersions, tensiolytes, oleolytes, tablets, pills, and gums, but preferably mouthwashes, gels and sprays, containing hyaluronic acid for the treatment of recurrent buccal aphthas.

Document ES 2343024 discloses a quilted, flexible adhesive bioabsorbable material containing collagen and antiseptics, anaesthetic and antimicrobials for the treatment of inflammation, wounds and/or aphthas in the buccal zone.

Aphthous stomatitis is treated with diverse oral formulations based on Triclosan and zinc salts (WO 94/26258) or with a mixture zinc salts and magnesium (WO 2010/092534).

### Summary of the invention

The amount and variety of preparations mentioned in the state of the art shows that research and development relative to cosmetic and pharmaceutical formulations related to buccal ulcerations is up-to-date and intense. Therefore, it is an important, live field open to new formulations, dressings or applications that provide advantages over the already known ones, accelerating the curative process or the symptomatology, or facilitating the application thereof.

A disadvantage of the aforementioned formulations is their lack of sufficient dwell time in the mouth, due to their easy elimination with the saliva, or by swallowing, to exert their therapeutical action. In other cases, such as the sticks, it is necessary to hold the formulation, and therefore place it in contact, with the fingers, which can cause in the final phase thereof ingestion of microorganisms where there is a lack of hygiene.

These disadvantages are particularly remarkable, accentuated and reasonably obvious when the formulation is used with children.

Concerning the above mentioned questions, after developed research work, fundamentally with respect to the improvement of the treatment of buccal ulcerations by means of simple, easily applicable pharmaceutical formulations and compositions, there is surprisingly proposed, for the first time, in this invention, a galenic formulation for buccal use that conjugates a pharmaceutical composition including, as active ingredients, effective doses of hyaluronic acid (and/or one of its pharmaceutically acceptable derivatives, such as for example one of its pharmaceutically acceptable salts) and a zinc compound, with a formulation and presentation in form of tablets with inserted stick, that allows the intermittent or continuous dwell of the preparation in contact with buccal ulcerations, the hygienic use of the preparation, and combining the attractiveness, for its form, flavour and aroma, of a lollypop form sweet or gum and, therefore facilitating the facility and desire of the children for the treatment. All these factors make said composition and presentation very advantageous in the treatment of buccal ulcers in children.

There is proposed, therefore, a new simple technical solution, in accordance with which, the inventive pharmaceutical product, unlike other types of formulations and applications such as gels, ointments, creams, sprays, solutions, etc. allows it to be easily applied in buccal ulcers, remaining in continuous or intermittent contact during the time required for its total dissolution and the consequent therapeutical action in the mouth; the product to be handled hygienically and it provides, in addition, security in its use in the treatment of children as having a stick that prevents it from being swallowed or choking.

A particularly advantageous application is in the treatment of buccal ulcers in children.

Therefore, an object of the present invention is to provide a tablet with stick containing pharmaceutical active ingredients and where said pharmaceutical active ingredients are hyaluronic acid or a pharmaceutically acceptable salt thereof and a zinc compound.

A further object of the present invention is a tablet with stick containing hyaluronic acid and/or a pharmaceutically acceptable derivative thereof and a zinc compound, to produce a pharmaceutical product useful in the treatment of buccal ulcers in children.

A further objective of the present invention is to disclose a therapeutical process including the application, by oral topical route of a pharmaceutical composition based on hyaluronic acid and/or its pharmaceutically acceptable derivatives and a zinc compound, by means of a tablet with stick.

### Brief description of the drawings

Further advantages and characteristics of the invention will become obvious from the following description, in which, without any limitative nature, preferred ways of embodiment of the invention are related, with reference to the accompanying drawings in which:

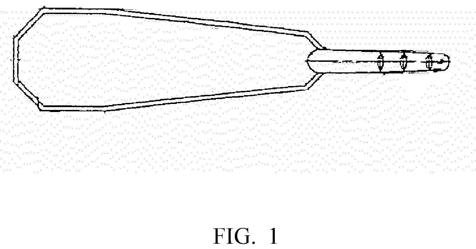

Figure 1 is a schematic view of a stick for a tablet according to the invention.

### Detailed description of the invention

The present invention relates to tablets with stick containing hyaluronic acid or pharmaceutically acceptable salts thereof and a zinc compound for use in the treatment of buccal ulcers in children.

According to the invention, further to their composition, the tablets are characterized by being supported on a stick or by having a stick as support.

The process for the preparation of the tablets with stick is characterized by initially preparing the pharmaceutical composition from the corresponding excipients and active ingredients, moulding the mass to give it the desired form and subsequently incorporating the stick while the mass is hot.

The pharmaceutical composition with which the tablets are formulated has a solid gel appearance containing the active ingredients and the different pharmaceutically acceptable excipients and adjuvants: thickeners, mucoadhesive agents, fillers, sweeteners, colouring agents, flavouring agents, solubilizing agents, etc. to obtain the required texture and pharmaco-technical properties.
Hyaluronic acid or salts thereof and zinc compounds are used as active ingredients.

The hyaluronic acid used is a hyaluronic acid of high molecular weight > 1 MDa, preferably between 1 and 5 MDa, and in the form of an alkali metal salt, preferably as sodium salt, at a concentration of between 0.05% and 5%, preferably between 0.1 % and 1%.

Among the zinc compounds the organic acid salts or complexes are described and among these the alphahydroxylated acids such as: glycolic, lactic, alphahydroxybutyric, alphahydroxypentanoic, alphahydroxyhexanoic acids and the like, anhydrous or hydrated zinc lactate at a concentration of between 0.05% and 5%, preferably between 0.1% and %. The zinc compound is dihydrated zinc lactate. Among the thickener excipients the ones of choice are gelatines and preferably gelatines with a thickening index ("bloom" degree or gel strength) comprised between 75 and 250 at a concentration of between 10% and 50%.
The sweeteners may be natural of polyhydroxylated type, alone or in combination of several of these or with artificial sweeteners. Preferred among the polyhydroxylated natural sweeteners are sugar, xylol, neohesperidine, sorbitol, mannitol, polydextrose, etc., which in turn can act as fillers. The total concentration may vary between 10% and 60%. Among the artificial sweeteners are sodium saccharin, sodium cyclamate, potassium acesulfame or the like, preferably in sodium salt form at a concentration of between 0.05% and 1.5%.

Among the mucoadhesives the homopolymeric derivatives of pyrrolidone, polyvinyl alcohols or polyacrylic acid derivatives are preferred. The homopolymeric derivatives of pyrrolidone at a concentration of between 0.1 % and 5%, preferably between 0.4% and 2%, are preferred.

Among the flavouring agents, any of the different food flavourings may be used, but strawberry, raspberry or orange flavouring agents are preferred.

Among the colouring agents artificial food colouring agents, alone or in combination, preferably of the azoic type such as amaranth, azorubine, tartrazine, orange yellow, etc., or natural such as cochineal, caramel, curcumin, orchil, red fruit colour, etc. may be used. Preferably those that provide red colours are used at a concentration of between 0.01% and 0.5% of pure colouring agent.

As preservatives the parabens, benzoic acid and salts thereof and sorbic acid and salts thereof may be used.

Among the solubilizers water and low molecular weight alcohols may be used, among which water and ethyl alcohol are preferred. Water will be always present in amounts of above 20% and below 45% and may vary based on the needs of adjustment of the formula. Ethyl alcohol could be absent or be used in amounts ranging from 0.5% to 5%.

The sticks inserted in the tablet may be made from different materials such as compressed paper or cardboard, plastic polymeric materials such as polythene, polypropylene, styrene, etc. Sticks made from materials such as polythene or HD polypropylene are preferred.

The sticks may be of cylindrical or flat shape, the flat shape being preferred for the external body of the stick (non-inserted portion) and cylindrical shape for the portion inserted therein. The cylindrical portion will contain, in addition notches facilitating the adherence thereof to the tablet. The flat portion may be moulded or not to incorporate the name of the product and may have different appearances and dimensions allowing it to be inserted in the tablet, the shape presented in Figure 1 being preferred, but not limiting.

The tablets may be flat, cylindrical or egg-shaped, with or without embossment or engraving, depending on the moulds used and the desired commercial presentation. The cylindrical shape, without embossment or engraving, with flat or rounded ends, is preferred. The weight of the tablets may be selected for the appropriate therapeutical dosage by adjustment of a volumetric dispenser containing the solution and the dimensions thereof will be adapted to the desired commercial presentation by means of the volume of the mould cavities. Dimensions of 1.5 to 2.5 centimetres length by 0.5 to 1.2 centimetres diameter are preferred for the cylindrical tablets.

According to the invention, the pharmaceutical composition forming the tablet with stick for its application in buccal ulcers in children is formulated as a solution that comprises all the previously mentioned components dissolved at a temperature of between 70 °C and 90 °C. This solution is placed under vacuum and then cooled to a temperature of between 55 °C and 65ºC prior to being poured into the corresponding aluminium or stainless steel moulds, independent for manual processing, or connected to a linear continuous automatic machine with stepwise transverse feed. After adjusting the mould temperature, filling is effected by volumetric metering. Once the solution has been metered in the moulds, the sticks may be introduced into the solution contained in the moulds simultaneously in several units, preferably three at a time. Subsequently the moulds are cooled in a cold chamber or cooling tunnel to temperatures of below 10°C.

Finally the product is stripped from the moulds by pressure of the corresponding extractors.

### Examples

Different formulas are given hereinafter as examples of the solution according to the invention, without being considered as limiting.

| Formula 1 | %p/p |
|---|---|
| Purified water | 20-35 |
| Hyaluronic acid sodium salt 1.3-1.8 MDa | 0.1-0.5 |
| Zinc lactate dihydrate | 0.1-0.5 |
| Nipagin M | 0.03-0.25 |
| Aqueous Cochineal | 5-20 |
| Xylitol C | 30-50 |
| Sodium Cyclamate | 0.1-0.8 |
| Gelatine | 20-30 |
| Strawberry flavouring | 0.1-1.2 |
| Total | 100 |

| Formula 2 | %p/p |
|---|---|
| Purified water | 20-35 |
| Hyaluronic acid sodium salt 1.3-1.8 MDa | 0.1-0.5 |
| Zinc lactate dihydrate | 0.1-0.5 |
| Plasdone | 0.2-3.0 |
| Nipagin M | 0.03-0.25 |
| Aqueous Cochineal | 5-20 |
| Polydextrose | 15-35 |
| Xylitol C | 10-20 |
| Sodium saccharin | 0.05-0.5 |
| Sodium Cyclamate | 0.1-0.8 |
| Gelatine | 15-25 |
| Strawberry flavouring | 0.1-1.2 |
| Total | 100 |

| Formula 3 | %p/p |
|---|---|
| Purified water | 30-40 |
| Hyaluronic acid sodium salt 1.3-1.8 MDa | 0.1-0.5 |
| Zinc lactate dihydrate | 0.1-0.5 |
| Ethanol | 0.5-5 |
| Nipagin M | 0.03-0.25 |
| Red fruit colouring | 0.02-0.2 |
| Sugar | 30-50 |
| Sodium saccharin | 0.05-0.5 |
| Gelatine | 25-40 |
| Strawberry flavouring | 0.5-1.5 |
| Total | 100 |

The stability studies carried out on the organoleptic properties, physicochemical properties (pH and viscosity) and hyaluronic acid and zinc content show long term stability of the pharmaceutical composition.

## Claims

1. - A pharmaceutical composition for topical application in the form of a tablet with stick, that comprises hyaluronic acid and/or one of the pharmaceutically acceptable derivatives thereof, and a zinc compound as active ingredients, **characterized in that** the zinc compound is dihydrated zinc lactate.

2. - A composition according to claim 1, **characterized in that** the hyaluronic acid and/or the pharmaceutically acceptable derivatives thereof have a molecular weight of above 1 MDa; preferably between 1 and 5 MDa.

3. - A composition according to one of claims 1 or 2, **characterized in that** the concentration of hyaluronic acid and/or the pharmaceutically acceptable derivatives, ranges from 0.05% to 0.5% by weight of the solution.

4. - A composition according to claim 1, **characterized in that** the concentration of the dihydrated zinc lactate ranges from 0.05% to 0.5% by weight of the solution.

5. - A composition according to anyone of claims 1 to 4, **characterized in that** the aqueous content is comprised between 20% and 45% by weight of the solution.

6. - A composition according to anyone of claims 1 to 5, **characterized in that** that the thickening agent is gelatine and ranges from 10% to 50% by weight of the solution.

7. - A composition according to anyone of claims 1 to 6, **characterized in that** it comprises mucoadhesives of the group formed by homopolymeric derivatives of pyrrolidone, polyvinyl alcohols or polyacrylic acid derivatives, and preferably it comprises homopolymeric derivatives of pyrrolidone at a concentration ranging from 0.1% to 5%; preferably from 0.4% to 2%.

8. - A tablet with stick having a composition according to anyone of claims 1 to 7.

9. - A tablet with stick according to claim 8 with of flat, cylindrical or egg shape having embossments or engravings or not.

10. - A tablet with stick according to claim 9 having a cylindrical shape without embossments or engravings.

11. - A tablet with stick according to anyone of claims 8 to 10 wherein the stick has a cylindrical or conical section suitable for being housed inside the tablet, and a flat section, preferably with a first triangular portion, close to said cylindrical or conical section, and a second rectangular portion.

12. - A process for the preparation of a pharmaceutical composition for topical application in the form of a tablet with stick including hyaluronic acid, and/or a pharmaceutically acceptable derivative, and a zinc compound as active ingredients, **characterized in that** it consists of preparing the tablet from a solution of all the components at a temperature of between 70ºC and 90°C that is deaerated by vacuum and cooled to be moulded at a temperature of between 55°C and 65ºC a nd inserting a stick at this temperature.

13. - A process according to claim 12 wherein after the stick is inserted in the tablet the moulds are cooled to a temperature of below 10 °C and the tablets are stripped from the mould at that temperature.

14. - The use of hyaluronic acid or a pharmaceutically acceptable derivative and a zinc compound, preferably zinc lactate, for the preparation of medicaments in the form of a tablet with stick for the topical treatment of buccal ulcers in children.

## Patentansprüche

1. Pharmazeutische Zusammensetzung zur topischen Anwendung in Form einer Tablette mit Stab, die Hyaluronsäure und/oder eines der pharmazeutisch akzeptablen Derivate davon und eine Zinkverbindung als Wirkstoffe umfasst, **dadurch gekennzeichnet, dass** die Zinkverbindung Zinklactat-Dihydrat ist.

2. Zusammensetzung gemäß Anspruch 1, **dadurch gekennzeichnet, dass** die Hyaluronsäure und/oder die pharmazeutisch akzeptablen Derivate davon ein Molekulargewicht von über 1 MDa, bevorzugt zwischen 1 und 5 MDa, aufweisen.

3. Zusammensetzung gemäß einem der Ansprüche 1 oder 2, **dadurch gekennzeichnet, dass** die Konzentration der Hyaluronsäure und/oder der pharmazeutisch akzeptablen Derivate im Bereich von 0,05 Gew.% bis 0,5 Gew.% der Lösung liegt.

4. Zusammensetzung gemäß Anspruch 1, **dadurch gekennzeichnet, dass** die Konzentration des Zinklactat-Dihydrats im Bereich von 0,05 Gew.% bis 0,5 Gew.% der Lösung liegt.

5. Zusammensetzung gemäß einem der Ansprüche 1 bis 4, **dadurch gekennzeichnet, dass** der Wassergehalt zwischen 20 Gew.% und 45 Gew.% der Lösung liegt.

6. Zusammensetzung gemäß einem der Ansprüche 1 bis 5, **dadurch gekennzeichnet, dass** das Verdickungsmittel Gelatine ist und im Bereich von 10 Gew.% bis 50 Gew.% der Lösung liegt.

7. Zusammensetzung gemäß einem der Ansprüche 1 bis 6, **dadurch gekennzeichnet, dass** sie Mucoadhäsive aus der Gruppe umfasst, die aus homopolymeren Derivaten von Pyrrolidon, Polyvinylalkoholen oder Polyacrylsäurederivaten gebildet wird, und sie bevorzugt homopolymere Derivate von Pyrrolidon in einer Konzentration im Bereich von 0,1% bis 5%, bevorzugt von 0,4% bis 2%, umfasst.

8. Tablette mit Stab mit einer Zusammensetzung gemäß einem der Ansprüche 1 bis 7.

9. Tablette mit Stab gemäß Anspruch 8 mit flacher, zylindrischer oder Eier-Form, die Prägungen oder Gravuren aufweist oder nicht.

10. Tablette mit Stab gemäß Anspruch 9 mit zylindrischer Form ohne Prägungen oder Gravuren.

11. Tablette mit Stab gemäß einem der Ansprüche 8 bis 10, wobei der Stab einen zylindrischen oder konischen Bereich, der geeignet ist, innerhalb der Tablette untergebracht zu werden, und einen flachen Bereich, bevorzugt mit einem ersten dreieckigen Teil nahe dem zylindrischen oder konischen Bereich und einem zweiten rechteckigen Teil, aufweist.

12. Verfahren zur Herstellung einer pharmazeutischen Zusammensetzung zur topischen Anwendung in Form einer Tablette mit Stab, die Hyaluronsäure und/oder ein pharmazeutisch akzeptables Derivat und eine Zinkverbindung als Wirkstoffe beinhaltet, **dadurch gekennzeichnet, dass** es aus dem Herstellen der Tablette aus einer Lösung aller Komponenten bei einer Temperatur zwischen 70°C und 90°C, die mittels Vakuum entlüftet wird und abgekühlt wird, um bei einer Temperatur zwischen 55°C und 65°C geformt zu werden, und dem Einführen des Stabs bei dieser Temperatur besteht.

13. Verfahren gemäß Anspruch 12, bei dem nach dem Einführen des Stabes in die Tablette die Formen auf eine Temperatur unter 10°C abgekühlt und die Tabletten bei dieser Temperatur aus der Form gelöst werden.

14. Verwendung von Hyaluronsäure oder eines pharmazeutisch akzeptablen Derivats und einer Zinkverbindung, bevorzugt Zinklactat, zur Herstellung von Medikamenten in Form einer Tablette mit Stab zur topischen Behandlung von bukkalen Geschwüren bei Kindern.

## Revendications

1. Composition pharmaceutique pour une application topique sous la forme d'une tablette avec un bâtonnet, qui comprend de l'acide hyaluronique et/ou l'un des dérivés pharmaceutiquement acceptables de celui-ci, et un composé de zinc comme ingrédients actifs, **caractérisée en ce que** le composé de zinc est le lactate de zinc dihydraté.

2. Composition selon la revendication 1, **caractérisée en ce que** l'acide hyaluronique et/ou les dérivés pharmaceutiquement acceptables de celui-ci ont un poids moléculaire au-dessus de 1 MDa ; préférentiellement entre 1 et 5 MDa.

3. Composition selon les revendications 1 ou 2, **caractérisée en ce que** la concentration de l'acide hyaluronique et/ou les dérivés pharmaceutiquement acceptables varie dans la gamme de 0.05% à 0.5 % en poids de la solution.

4. Composition selon la revendication 1, **caractérisée en ce que** la concentration du lactate de zinc dihydraté varie dans la gamme de 0.05 % à 0.5 % en poids de la solution.

5. Composition selon les revendications 1 à 4, **caractérisée en ce que** le contenu aqueux est compris entre 20 % et 45 % en poids de la solution.

6. Composition selon les revendications 1 à 5, **caractérisée en ce que** l'agent épaississant est la gélatine et varie dans la gamme de 10% à 50% en poids de la solution.

7. Composition selon les revendications 1 à 6 ; **caractérisée en ce qu'**elle comprend des mucoadhésifs du groupe formé par les dérivés homopolymériques de pyrrolidone, I dérivés d'alcool polyvinylique ou les dérivés d'acide polyacrylique, et préférentiellement elle comprend des dérivés homopolymerique de pyrrolidone à une concentration variant dans la gamme de 0.1 % à 5 % ; de préférence de 0.4 % à 2%.

8. Tablette avec un bâtonnet ayant une composition selon les revendications 1 à 7.

9. Tablette avec un bâtonnet selon la revendication 8 avec un forme plate, cylindrique ou d'oeuf avec ou non des bosses ou des gravures.

10. Tablette avec un bâtonnet selon la revendication 9 ayant une forme cylindrique sans bosses ni gravures

11. Tablette avec un bâtonnet selon les revendications 8 à 10 dans laquelle le bâtonnet a une section cylindrique ou conique appropriée pour être placé dans la tablette et une section plane, préférentiellement avec une première portion triangulaire, proche de ladite section cylindrique ou conique, et une seconde section rectangulaire

12. Procédé pour la préparation d'une composition pharmaceutique pour une application topique sous la forme d'une tablette avec un bâtonnet incluant de l'acide hyaluronique et/ou des dérivés pharmaceutiquement acceptables, et un composé de zinc comme ingrédients actifs, **caractérisé en ce qu'**il consiste à préparer la tablette à partir d'une solution comprenant tous les composants à une température comprise entre 70°C et 90°C qui est désaérée par le vide et refroidie pour être moulée à une température comprise entre 55°C et 65°C et à insérer le bâtonnet à cette température.

13. Procédé selon la revendication 12 dans lequel après que le bâtonnet est inséré dans la tablette, les moules sont refroidis à une température de moins de 10°C et les tablettes sont retirées du moule à cette température.

14. Utilisation d'acide hyaluronique ou d'un dérivé pharmaceutiquement acceptable et d'un composé de zinc, de préférence du lactate de zinc, pour la préparation d'un médicament sous la forme d'une tablette avec un bâtonnet pour le traitement topique des ulcères buccaux chez les enfants.
